Europäisches Patentamt

European Patent Office

Office européen des brevets

(19)

(11) **EP 0 583 438 B1**

(12) **EUROPÄISCHE PATENTSCHRIFT**

(45) Veröffentlichungstag und Bekanntmachung des Hinweises auf die Patenterteilung:
**26.08.1998** Patentblatt 1998/35

(21) Anmeldenummer: **93902192.9**

(22) Anmeldetag: **14.01.1993**

(51) Int Cl.⁶: **C11B 11/00**, A61K 7/48, A61K 7/06

(86) Internationale Anmeldenummer:
**PCT/EP93/00067**

(87) Internationale Veröffentlichungsnummer:
**WO 93/17083 (02.09.1993 Gazette 1993/21)**

(54) **VERFAHREN ZUR GEWINNUNG VON APFELWACHS, DURCH DIESE VERFAHREN ERHÄLTLICHES APFELWACHS SOWIE APFELWACHSHALTIGE KOSMETISCHE MITTEL**

APPLE WAX EXTRACTION PROCESS, APPLE WAX THUS OBTAINED AND APPLE WAX-CONTAINING COSMETIC PRODUCTS

PROCEDE D'EXTRACTION DE CIRE DE POMMES, CIRE DE POMMES AINSI OBTENUE ET PRODUITS COSMETIQUES CONTENANT DE LA CIRE DE POMMES

(84) Benannte Vertragsstaaten:
**DE ES FR GB IT**

(30) Priorität: **28.02.1992 DE 4206154**

(43) Veröffentlichungstag der Anmeldung:
**23.02.1994** Patentblatt 1994/08

(73) Patentinhaber: **Wella Aktiengesellschaft**
**64295 Darmstadt (DE)**

(72) Erfinder:
 • **LANG, Günther, Dr.**
  **D-64354 Reinheim (DE)**
 • **HOCH, Dieter**
  **D-64319 Pfungstadt-Eich (DE)**
 • **KONRAD, Eugen**
  **D-64297 Darmstadt (DE)**
 • **GEIBEL, Wolfram, Dr.**
  **D-36088 Hünfeld (DE)**
 • **WENDEL, Harald**
  **D-64372 Ober-Ramstadt (DE)**
 • **KRIPP, Thomas, Dr.**
  **D-64407 Fränkisch-Crumbach (DE)**

(56) Entgegenhaltungen:
**DD-A- 287 871**

 • **FOOD SCIENCE & TECHNOLOGY ABSTRACTS no. 79-11-H1846 (79061736) ZAIKO, G.M. : "By-products from waste from apple juice manufacture" By-products from waste from apple juice manufacture" & KONSERVNAYA I OVOSHCHESUSHIL'NAYA PROMYSHLNNOST' No. 10, 1978, pages 39 - 40**
 • **LEBENSMITTEL-WISSENSCHAFT UND TECHNOLOGIE Bd. 19, Nr. 6, 1986, LONDON, GB Seiten 493 - 496 E. BUNDSCUH ET AL. 'Gewinnung von nat rlichen Aromen aus Reststoffen der Lebensmittelproduktion mit Hilfe der CO2-Hochdruckextraktion'**
 • **PATENT ABSTRACTS OF JAPAN vol. 11, no. 43 (C-402)(2490) 7. Februar 1987 & JP,A,61 207 321 ( ASAMI KASEI K.K. ) 13 September 1986**
 • **PATENT ABSTRACTS OF JAPAN vol. 13, no. 515 (C-655)(3863) 17. November 1989 & JP,A,1 207 218 ( DAIICHI ENG K.K. ) 21 August 1989**
 • **DATABASE WPI Week 7822, Derwent Publications Ltd., London, GB; AN 78-39536A & JP,A,53 044 639 (OGAWA Y) 21 Aril 1978**
 • **JOURNAL OF THE SCIENCE OF FOOD AND AGRICULTURE. Bd. 24, Nr. 11, 1973, BARKING GB Seiten 1331 - 1339 ISOBEL M. MORICE ET AL. 'Composition of the surface waxes of apple fruits and changes during storage'**

**Beschreibung**

Gegenstand der vorliegenden Erfindung sind Apfelwachs sowie kosmetische Mittel mit einem Gehalt an Apfelwachs und Verfahren zur Gewinnung von Apfelwachs.

In kosmetischen Mitteln können Wachse vielfältige Funktionen erfüllen. Beispielsweise können sie für sich alleine zur Abdeckung der Haut verwendet werden oder als Fettkomponente in Emulsionen zur Viskosität und Stabilität des kosmetischen Mittels beitragen. In hautkosmetischen Mittel eingesetzt, können Wachse der Rückfettung und Hydrophobierung der Haut dienen und in haarkosmetischen Mitteln eine Konditionierung und Pflege des Haares bewirken.

Aufgrund der Herstellungsweise kann man die Wachse in natürliche, chemisch veränderte natürliche und synthetische Wachse einteilen. Zu den für die Kosmetik bedeutsamen Wachsen gehören die Mineralwachse und bestimmte natürliche Wachse tierischer oder pflanzlicher Herkunft.

Natürliche und chemisch veränderte natürliche Mineralwachse stammen aus nicht erneuerbaren Rohstoffquellen, bzw. werden aus durch diese erzeugten Rohstoffen hergestellt. Synthetische Mineralwachse sind häufig mit synthesebedingten Verunreinigungen belastet.

Die Verwendung von natürlichen oder chemisch veränderten natürlichen Wachsen tierischer oder pflanzlicher Herkunft vermeidet die zuvor genannten Nachteile der Mineralwachse und kommt zudem den Wünschen weiter Verbraucherkreise nach der Verwendung von natürlichen, ökologisch unbedenklichen, d.h. nachwachsenden Rohstoffen entgegen.

Das bekannteste tierische Wachs ist das Bienenwachs, dessen Verfügbarkeit als Ausscheidungsprodukt der Bienen jedoch begrenzt ist. Darüberhinaus enthält das Bienenwachs hohe Anteile an Estern, was die Stabilität bienenwachshaltiger kosmetischer Mittel nachteilig beeinflussen kann.

Die derzeit am häufigsten in kosmetischen Mitteln eingesetzten, kommerziell erhältlichen pflanzlichen Wachse sind das Candelilla- und das Carnaubawachs. Diese Wachse sind jedoch hart und spröde, haben einen hohen Schmelzpunkt und sind in üblichen kosmetischen Mitteln nur schlecht emulgierbar.

Candelilla- und Carnaubawachs werden von der Oberfläche tropischer Palmenblätter gewonnen, deren Wachsschicht besonders dick ist, da sich die Pflanze im heißen tropischen Klima gegen den Verlust von Wasser stärker schützen muß als Pflanzen, die in gemäßigten Klimazonen heimisch sind. Aufgrund der vergleichsweise geringen Menge an Oberflächenwachs der in gemäßigten Klimazonen wachsenden Pflanzen, erscheint die Isolierung der Oberflächenwachse heimischer Pflanzen als unwirtschaftlich, obwohl diese Pflanzen in großen Mengen zur Verfügung stehen.

Es bestand daher die Aufgabe, ein Oberflächenwachs einer in gemäßigten Klimazonen heimischen Pflanze, das die zuvor beschriebenen Nachteile der in kosmetischen Mitteln üblicherweise eingesetzten Wachse nicht aufweist und kosmetische Mittel mit einem Gehalt an diesem Oberflächenwachs zur Verfügung zu stellen.

Diese Aufgabe wird durch ein Apfelwachs gelöst, das einen Tropfpunkt von 55°C oder niedriger aufweist sowie durch kosmetische Mittel, die dieses Apfelwachs enthalten, und durch Verfahren zur Gewinnung des Apfelwachses.

Gegenstand der vorliegenden Erfindung ist daher auch ein Verfahren zur Gewinnung von Apfelwachs, bei dem man

a) entpektinisierten Apfeltrester mit Äthanol extrahiert,

b) den Extrakt zur Trockene einengt, den erhaltenen Rückstand bei einem pH-Wert von mindestens 8 mit einem Gemisch aus gleichen Teilen Essigsäureethylester und Wasser extrahiert,

c) anschließend die organische Phase einengt und das Apfelwachsrohprodukt mit t-Butylmethylether, Wasser und Bleicherde in der Siedehitze behandelt, den t-Butylmethylether abdestilliert, den Rückstand abfiltriert und trocknet,

d) den getrockneten Rückstand mit t-Butylmethylether extrahiert und den Extrakt zur Trockene einengt.

Die Einstellung des pH-Wertes auf mindestens 8 erfolgt mit Alkalien, bspw. mit Natronlauge, Calciumhydroxid, Natriumcarbonat oder Kaliumcarbonat.

Das nach dem vorstehenden Verfahren erhältliche Apfelwachs kann durch erneutes Auflösen in siedendem Petroläther und eine weitere Behandlung mit Bleicherde noch weiter gereinigt werden.

Nach dem vorstehend beschriebenen erfindungsgemäßen Verfahren wird hellgelbes, geruchloses Apfelwachs mit einem Tropfpunkt von 55° C oder niedriger erhalten.

Die in den erfindungsgemäßen Verfahren verwendete Bleicherde kann durch Cellulosepulver, bspw. in Form des Handelsproduktes Arbocel® der Firma J. Rettenmaier & Söhne GmbH & Co., Holzmühle, BRD, ersetzt werden.

In einer besonderen Ausführungsform der erfindungsgemäßen Verfahren wird der entpektinisierte Apfeltrester vor der Extraktion gemahlen.

Zum Mahlen kleinerer Mengen entpektinisierten Apfeltresters kann bspw. eine IKA-Universalmühle M20 der Firma Janke und Kunkel Ika Werk, Staufen, BRD, verwendet werden. Der gemahlene entpektinisierte Apfeltrester kann zur Gewährleistung einer einheitlichen Korngröße nach dem Mahlschritt ggf. noch mit einem handelsüblichen Haushaltssieb gesiebt werden.

Das erfindungsgemäße Verfahren zur Gewinnung von Apfelwachs erlaubt eine wirtschaftliche Gewinnung des Oberflächenwachses der Äpfel.

Das Ausgangsprodukt, entpektinisierter Apfeltrester, ist als Abfallprodukt der Pektin herstellenden Industrie billig verfügbar. Die Ausbeute an Apfelwachs aus entpektinisiertem Apfeltrester ist gegenüber der Verwendung pektinhaltigen Apfeltresters zur Gewinnung von Apfelwachs signifikant erhöht. Diese Ausbeutenerhöhung ist überraschend, da das bei dem üblichen Verfahren zur Pektingewinnung aus Apfeltrester, wie es bspw. in Ullmanns Encyklopädie der technischen Chemie, Band 19, 1980, Seiten 239 - 240 beschrieben wird, verwendete angesäuerte Wasser über den Schmelzpunkt des Apfelwachses hinaus erwärmt wird.

Das verwendete Extraktionsmittel Ethanol ist toxikologisch unbedenklich, so daß der Tresterrückstand, z.B. als Viehfutter, weiterverwendet werden kann.

Zudem können die für die erfindungsgemäßen Verfahren verwendeten organischen Lösungsmittel, ohne daß Ausbeuteverluste an Apfelwachs auftreten, bis zu 50 Gewichtsprozent Wasser enthalten. Die nach der wäßrigen Extraktion des Pektins aus Apfelvolltrester bisher übliche Trocknung des anfallenden entpektinisierten Apfeltresters kann daher bei der Weiterverwertung des entpektinisierten Tresters in dem erfindungsgemäßen Verfahren zur Gewinnung des Apfelwachses entfallen. Neben der vorteilhaften Energieeinsparung durch den Wegfall des Trocknungsschrittes, ist das Apfelwachs aus nicht getrocknetem entpektinisierten Apfeltrester zudem nicht mit Karamelisierungsprodukten verunreinigt, die durch diesen Trocknungsschritt entstehen können.

Das erfindungsgemäße Apfelwachs zeigt eine hautschützende Wirkung und weist einen vergleichbaren okklusiven Effekt auf wie die häufig in kosmetischen Mitteln eingesetzte Vaseline. Apfelwachs ist daher als Bestandteil hautschützender und hautpflegender kosmetischer Mittel hervorragend geeignet.

Apfelwachs kann in reiner Form zur Abdeckung und zum Schutz der Haut verwendet werden. Als Bestandteil kosmetischer Haarpflege- und Haarreinigungsmittel verbessert Apfelwachs die Naß- und Trockenkämmbarkeit sowie den Griff des Haares.

Mit dem erfindungsgemäßen Apfelwachs lassen sich daher kosmetische Mittel zur Behandlung der Haut und der Haare herstellen, die sich durch überraschend vorteilhafte Eigenschaften auszeichnen. Mittel zur Behandlung der Haut und der Haare mit einem Gehalt an Apfelwachs sind daher ebenfalls Gegenstand der vorliegenden Anmeldung.

Die erfindungsgemäßen Mittel können bspw. als Mittel zur Pflege oder zum Schutz der Haut, wie Tagescremes, Nachtcremes, Hautcremes, Sonnenschutzcremes, Lippenstifte, Lippenpflegemittel oder auch Make up-Präparate wie Schminkcremes oder Rouge; als Haar- und/oder Körperreinigungsmittel, wie Shampoos, Duschbäder, Schaumbäder oder Reinigungslotionen; als Haarbehandlungsmittel wie Haaröle, Brillantinen oder Haarwachse oder als Haarpflegemittel, wie Haarkuren und Haarspülungen, vorliegen.

Wenn es sich bei dem erfindungsgemäßen Mittel um ein emulsionsförmiges Hautschutz- oder Hautpflegemittel handelt, enthält das Mittel bevorzugt 0,05 bis 50 Gewichtsprozent Apfelwachs.

Ein in Form einer Öl-in-Wasser-Emulsion vorliegendes erfindungsgemäßes Hautschutz- oder Hautpflegemittel enthält besonders bevorzugt 0,05 bis 20 Gewichtsprozent Apfelwachs.

Ein in Form einer Wasser-in-Öl-Emulsion vorliegendes Hautschutz- oder Hautpflegemittel enthält besonders bevorzugt 1 bis 50 Gewichtsprozent Apfelwachs.

Handelt es sich bei dem erfindungsgemäßen Mittel um ein Haarund/oder Körperreinigungsmittel oder um ein Haarpflegemittel, weist es bevorzugt einen Gehalt an 0,5 bis 2 Gewichtsprozent Apfelwachs auf.

Erfindungsgemäße Sonnenöle enthalten bevorzugt 0,1 bis 80 Gewichtsprozent Apfelwachs. Erfindungsgemäße Haaröle, Brillantinen und Haarwachse enthalten bevorzugt 1 bis 99,8 Gewichtsprozent Apfelwachs.

Die Zusammensetzung der erfindungsgemäßen Mittel kann eine Mischung aus Apfelwachs, mit den für solche Zubereitungen üblichen physiologisch verträglichen Bestandteilen, wie Träger- und Zusatzstoffen, darstellen.

Das erfindungsgemäße Mittel kann in einer besonderen Ausführungsform ausschließlich aus Apfelwachs bestehen und in dieser Form zur Abdeckung und zum Schutz der Haut oder als Haarwachs eingesetzt werden.

Das erfindungsgemäße Mittel kann in einer beliebigen für Haut und Haarbehandlungsmittel geeigneten Zubereitungsform, bspw. in Form einer wäßrig-alkoholischen oder alkoholischen Lösung, als Emulsion, als Creme oder als Gel vorliegen. Das Mittel kann auch im Gemisch mit einem Treibgas oder mittels einer Pumpe versprüht werden.

Übliche Zusätze in Lösungen, Cremes, Emulsionen oder Gelen sind, neben Lösungsmitteln wie Wasser und niederen aliphatischen Alkoholen, bspw. Ethanol, Propanol und Isopropanol oder Glykolen wie Glycerin und 1,2-Propylenglykol; Netzmittel oder Emulgatoren aus den Klassen der anionischen, kationischen, amphoteren oder nichtionogenen oberflächenaktiven Substanzen, wie Fettalkoholsulfate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, in einer Menge von 0,1 bis 30 Gewichtsprozent; Parfümöle in einer Menge von 0,5 bis 5,0 Gewichtsprozent; Trübungsmittel, wie z.B. Ethylenglykoldistearat, in einer Menge von etwa 0,5 bis 5,0 Gewichtsprozent; Perlglanzmittel, wie z.B.

ein Gemisch aus Fettsäuremonoalkylolamid und Ethylenglykoldistearat, in einer Menge von etwa 1,0 bis 10 Gewichtsprozent; bakterizide und fungizide Wirkstoffe, wie z .B. 2,4,4-Trichlor-2-hydroxydiphenylether oder Methylchlorisothiazolinon, in einer Menge von 0,01 bis 1,0 Gewichtsprozent; Verdickungsmittel, wie bspw. Kokosfettsäuzrediethanolamid, in einer Menge von etwa 0,5 bis 3,0 Gewichtsprozent; Puffersubstanzen wie bspw. Natriumcitrat oder Natriumphosphat, in einer Menge von 0,1 bis 1,0 Gewichtsprozent; Lösungsvermittler, wie z.B. ethoxyliertes Rizinusöl, in einer Menge von etwa 0,1 bis 1,0 Gewichtsprozent; Anfärbestoffe wie z.B. Fluorescein-Natriumsalz in einer Menge von etwa 0,1 bis 1,0 Gewichtsprozent; Pflegestoffe wie z.B. kationische Harze, Lanolinderivate, natürliche, chemisch veränderte natürliche oder synthetische Wachse und Öle wie Mandelöl, Jojobaöl, Bienenwachs, Walrat, in einer Menge von 0,1 bis 5 Gewichtsprozent, sowie ferner Lichtschutzmittel, Feuchthaltemittel, Antioxidantien, Komplexbildner und Antischuppenwirkstoffe in einer Menge von etwa 0,01 bis 0,8 Gewichtsprozent.

Die nachfolgenden Beispiele sollen den Gegenstand der Erfindung näher erläutern.

**Beispiel 1: Gewinnung von Apfelwachs**

100 g entpektinisierter Apfeltrester werden in 400 ml absolutem Ethanol 1 Stunde lang unter Rückfluß erhitzt und heiß filtriert. Der Extrakt wird zur Trockene eingeengt. Man erhält 13,8 g eines Rohproduktes, das in 276 ml Essigsäureethylester und 276 ml Wasser aufgenommen wird. Die Lösung wird mit Natriumcarbonat auf pH = 10 eingestellt, 10 Minuten lang unter Rückfluß erhitzt und sodann auf Raumtemperatur abgekühlt. Die Essigsäureethylesterphase wird anschließend abgetrennt und zur Trockene eingeengt.

Das als Rückstand der Essigsäureethylesterphase verbliebene Apfelwachsrohprodukt wird mit 0,3 1 t-Butylmethylether, 0,14 1 Wasser und 30 g Bleicherde 1 Stunde lang unter Rückfluß erhitzt. Sodann wird der t-Butylmethylether abdestilliert, der Rückstand aus der verbliebenen Wasserphase abfiltriert und im Vakuum bei 40° C getrocknet. Der getrocknete Rückstand wird in 300 ml t-Butylmethylether 1 Stunde lang unter Rückfluß erhitzt. Sodann wird filtriert und durch das Enengen des Filtrats zur Trockene werden 4,2 g Apfelwachs erhalten.

**Beispiel 2: Reinigung von Apfelwachs**

600 g nach Beispiel 1 gewonnenes Apfelwachs werden in 18 1 Petrolether zum Sieden erhitzt und in der Hitze filtriert. Das Filtrat wird auf Raumtemperatur abgekühlt, mit 1100 g Bleicherde versetzt und 1 Stunde lang gerührt. Sodann wird die Bleicherde abfiltriert und das Filtrat zur Trockene eingeengt. Man erhält 230 g geruchloses, hellgelbes Apfelwachs mit einem Tropfpunkt von 54,8 Grand Celsius.

**Beispiele für kosmetische Mittel**

| Beispiel I: | Hautcreme mit schützender Wirkung |
|---|---|
| 2,00 g | Apfelwachs |
| 8,00 g | Glycerinstearat, selbstemulgierend |
| | eines Gemisches aus Glycerylhydroxystearat, |
| | Cetylpalmitat, mikrokristallinem Wachs und |
| | Trihydroxystearin |
| 1,00 g | Stearin |
| 8,00 g | Paraffinium perliquidum |
| 0,10 g | p-Hydroxybenzoesäurepropylester |
| 0,20 g | p-Hydroxybenzoesäuremethylester |
| 0,05 g | Allantoin |
| 0,20 g | Parfüm |
| 78,45 g | Wasser |
| 100,00 g | |

| Beispiel II: | Hautcreme mit schützender Wirkung |
|---|---|
| 4,00 g | Apfelwachs |
| 8,00 g | Glycerinstearat, selbstemulgierend |

(fortgesetzt)

| Beispiel II: | Hautcreme mit schützender Wirkung |
|---|---|
| 1,00 g | Stearin |
| 8,00 g | flüssiges Paraffin |
| 0,10 g | p-Hydroxybenzoesäurepropylester |
| 0,20 g | p-Hydroxybenoesäuremethylester |
| 0,05 g | Allantoin |
| 0,20 g | Parfüm |
| 78,45 g | Wasser |
| 100,00 g | |

**Beispiel III: Vergleichsversuche zur Hautschutzwirkung**

Auf je eine 2 cm$^2$ große Hautfläche der rechten Unterarminnenseiten von 10 Versuchsteilnehmern wurde die Hautcreme gemäß Beispiel I und auf eine weitere 2 cm$^2$ große Hautfläche des gleichen Unterarms die Hautcreme gemäß Beispiel II aufgetragen und 1 Minute lang leicht eingerieben. Sodann wurde der Überschuß an Hautcreme durch leichtes Tupfen mit Zellstoff entfernt. Die linken Unterarmseiten der Versuchsteilnehmer blieben zunächst unbehandelt.

Mit einem IR-Strahlungsthermometer KT 15 der Firma Heimann GmbH, Wiesbaden, Objetiv L, wurde bei gleicher Distanz des Objektivs zur Hautoberfläche und konstanter Umgebungstemperatur die Hauttemperatur der mit den Hautcremes gemäß Beispiel I und II behandelten beiden Hautflächen des rechten Unterarms und einer unbehandelten Hautfläche des linken Unterarms gemessen.

Sodann wurde eine 4%ige wäßrige Ammoniaklösung auf die jeweils 2 cm$^2$ großen, mit den Hautcremes gemäß Beispiel I beziehungsweise Beispiel II behandelten Hautflächen sowie auf eine gleichgroße bisher unbehandelte Fläche des linken Unterarms der Versuchsteilnehmer aufgetragen. Nach 10 Minuten wurde dann die Hauttemperatur in der zuvor beschriebenen Weise erneut bestimmt. Aus den an den 10 Versuchsteilnehmern gemessenen Temperaturen der rechten und der linken Unterarme vor und nach der Behandlung mit Ammoniak wurden sodann Mittelwerte der Temperaturerhöhungen errechnet und in der folgenden Tabelle zusammengestellt.

| Behandlung mit | Mittelwert der Hauttemperaturerhöhung |
|---|---|
| 1. Hautcreme nach Beispiel I <br><br> 2. Ammoniaklösung | 1,06 ± 1,38°C |
| Kontrolle <br><br> 1. Ammoniaklösung | 1,33 ± 0,75°C |
| 1. Hautcreme nach Beispiel II <br><br> 2. Ammoniaklösung | 1,14 ± 1,22°C |
| Kontrolle <br><br> 1. Ammoniaklösung | 1,30 ± 0,73°C |

Die Vergleichsversuche zeigen, daß die vorherige Anwendung der Hautcremes gemäß den Beispielen I und II die durch Ammoniak bewirkte Hauttemperaturerhöhung verringern und somit eine Hautschutzwirkung aufweisen.

| Beispiel IV | Shampoo |
|---|---|
| 11,20 g | Gemisch aus Natriumlaurylethersulfat und Salicylsäure |
| 1,50 g | Triglykoldistearat |
| 0,50 g | Apfelwachs, hergestellt nach Beispiel 1 |
| 4,20 g | Natriumchlorid |

(fortgesetzt)

| Beispiel IV | Shampoo |
|---|---|
| 82,60 g | Wasser |
| 100,00 g | |

Das Shampoo ergibt einen seidigen Schaum und verbessert die Kämmbarkeit und den Griff des damit gewaschenen Haares

| Beispiel V | Haarkurmittel |
|---|---|
| 3,500 g | Cetylstearylalkohol |
| 0,500 g | Apfelwachs, hergestellt nach Beispiel 1 |
| 0,550 g | Cetyltrimethylammoniumchlorid |
| 0,004 g | Anfärbefarbstoff |
| 0,200 g | Pflanzenextrakt (Extrapon 5 Spezial) |
| 0,500 g | Zitronensäure |
| 0,900 g | Parfümöl |
| 93,846 g | Wasser |
| 100,000 g | |

Das Haarkurmittel läßt sich problemlos nach der Anwendung aus dem Haar spülen, ergibt eine gute Naß- und Trockenkämmbarkeit und verleiht dem Haar einen guten Griff und ein gepflegtes Aussehen.

| Beispiel VI | Haarkurmittel |
|---|---|
| 2,360 g | Cetylstearylalkohol |
| 0,900 g | Laurylalkoholdiglykolether |
| 1,240 g | Vaseline |
| 1,000 g | Apfelwachs |
| 10,000 g | Betainmonohydrat |
| 0,378 g | Cetylstearylsulfat-Natriumsalz |
| 0,150 g | D,L-Mandelsäure |
| 0,150 g | Salicylsäure |
| 2,000 g | Zitronensäure |
| 0,300 g | Parfüm |
| 81,522 g | Wasser |
| 100,000 g | |

Das Haarkurmittel läßt problemlos nach der Anwendung aus dem Haar spülen, ergibt eine gute Naß- und Trockenkämmbarkeit und verleiht dem Haar einen guten Griff und ein gepflegtes Aussehen.

| Beispiel VII | Lippenpflegestift |
|---|---|
| 25,00 g | Rizinusöl |
| 5,00 g | Jojobaöl |
| 20,00 g | Mineralöl |
| 25,00 g | mikrokristallines Wachs |
| 15,00 g | Decyloleat |
| 8,00 g | Apfelwachs |
| 1,70 g | Phenyltrimethicone |
| 0,30 g | Parfüm |
| 100,00 g | |

| Beispiel VIII | Sonnenschutzcreme (Öl-in-Wasser-Emulsion) |
|---|---|
| 8,00 g | Glycerylstearat |
| 2,00 g | Cetyl-Stearylalkohol |
| 1,50 g | Cetyl-Stearylalkohol, mit 20 Mol Ethylenoxid oxethyliert |
| 1,50 g | Cetyl-Stearylalkohol, mit 12 Mol Ethylenoxid oxethyliert |
| 3,00 g | Apfelwachs |
| 6,00 g | gemischte Triester des Glycerins mit Caprinsäure und Caprylsäure |
| 8,00 g | Gemisch der Ester der Caprinsäure und Caprylsäure mit Kokosnußalkolhol |
| 6,00 g | Dibutyladipat |
| 4,00 g | Octyldodecanol |
| 3,00 g | Dimethylpolysiloxan |
| 4,00 g | Lichtschutzfilter |
| 2,58 g | Glycerin |
| 50,42 g | Wasser |
| 100,00 g | |

Sämtliche in dieser Anmeldung angegebenen Prozentzahlen stellen Gewichtsprozente dar.

**Patentansprüche**

1. Apfelwachs, **dadurch gekennzeichnet**, daß es einen Tropfpunkt von 55° C oder niedriger aufweist.

2. Kosmetisches Mittel zur Behandlung der Haut oder der Haare, **dadurch gekennzeichnet**, daß es ein Apfelwachs gemäß Anspruch 1 enthält.

3. Kosmetisches Mittel nach Anspruch 2, **dadurch gekennzeichnet**, daß es in Form einer Lösung, einer Emulsion, einer Creme oder eines Gels vorliegt.

4. Kosmetisches Mittel nach Anspruch 3, **dadurch gekennzeichnet**, daß es ein emulsionsförmiges Hautschutz- oder Hautpflegemittel mit einem Gehalt von 0,05 bis 50 Gew.-% eines Apfelwachses gemäß Anspruch 1 ist.

5. Kosmetisches Mittel nach Anspruch 3, **dadurch gekennzeichnet**, daß es ein Haar- oder Körperreinigungsmittel oder ein Haarpflegemittel mit einem Gehalt von 0,5 bis 2,0 Gew.-% eines Apfelwachses gemäß Anspruch 1 ist.

6. Verfahren zur Gewinnung eines Apfelwachses nach Anspruch 1, **dadurch gekennzeichnet**, daß man

   a) entpektinisierten Apfeltrester mit Ethanol extrahiert,
   b) den Extrakt sodann zur Trockene einengt, den erhaltenen Rückstand bei einem pH-Wert von mindestens 8 mit einem Gemisch aus gleichen Teilen Essigsäureethylester und Wasser extrahiert,
   c) anschließend die organische Phase einengt und das Apfelwachsrohprodukt mit t-Butylmethylether, Wasser und Bleicherde in der Siedehitze behandelt, den t-Butylmethylether abdestilliert, den Rückstand abfiltriert und trocknet,
   d) den getrockneten Rückstand mit t-Butylmethylether extrahiert und den Extrakt zur Trockene einengt.

**Claims**

1. Apple wax, characterised by a melting point of 55°C or lower.

2. Cosmetic composition for the treatment of the skin or the hairs, characterised by its content of the apple wax of claim 1.

3. Cosmetic composition of claim 2, which is in the form of a solution, an emulsion, a creme or a gel.

4. Cosmetic composition of claim 3, which is an emulsion-like skin protection or skin care preparation containing 0,05 up to 50 percent by weight of the apple wax of claim 1.

5. Cosmetic composition of claim 3, which is a hair or body cleansing preparation or a hair care preparation containing 0,5 up to 2,0 percent by weight of the apple wax of claim 1.

6. Process for the preparation of the apple wax of claim 1, characterised in that

    a) depectined apple residues are extracted with ethanol,
    b) the extract is thereafter concentrated up to dryness, the residue is extracted at a pH of at least 8 with a mixture of equal parts of acetic acid ethyl ester and water,
    c) thereafter the organic phase is concentrated and the raw apple wax is treated with t-butylmethylether, water and bleaching earth at the boiling temperature, the t-butylmethylether is distilled away, the residue is separated by filtration and dried,
    d) the dried residue is extracted with t-butylmethylether and the extract is concentrated up to dryness.

**Revendications**

1. Cire de pommes, caractérisée en ce qu'elle présente un point de goutte inférieur ou égal à 55°C.

2. Produit cosmétique de traitement de la peau ou des cheveux, caractérisé en ce qu'il contient une cire de pommes selon la revendication 1.

3. Produit cosmétique selon la revendication 2, caractérisé en ce qu'il se présente sous la forme d'une solution, d'une émulsion, d'une crème ou d'un gel.

4. Produit cosmétique selon la revendication 3, caractérisé en ce qu'il s'agit d'un produit de protection ou de soin de la peau sous forme d'émulsion ayant une teneur de 0,05 à 50 % en poids de cire de pommes selon la revendication 1.

5. Produit cosmétique selon la revendication 3, caractérisé en ce qu'il s'agit d'un shampoing capillaire ou corporel ou d'un produit de soin capillaire ayant une teneur de 0,5 à 2,0 % en poids de cire de pommes selon la revendication 1.

6. Procédé d'obtention d'une cire de pommes selon la revendication 1, caractérisé en ce que

    a) on extrait à l'éthanol du marc de pommes dépectiné,
    b) on concentre immédiatement l'extrait à siccité, on extrait le résidu obtenu à un pH d'au moins 8 avec un mélange en parties égales d'acétate d'éthyle et d'eau,
    c) on concentre ensuite la phase organique et on traite le produit brut de cire de pommes avec du t-butylméthyléther, de l'eau et une argile décolorante à la température d'ébullition, on chasse le t-butylméthyléther par distillation, on sépare le résidu par filtration et on le sèche,
    d) on extrait le résidu séché avec du t-butylméthyléther et on concentre l'extrait à siccité.